# EUROPÄISCHE PATENTSCHRIFT

(11) **EP 0 930 078 B1**
(45) Veröffentlichungstag und Bekanntmachung des Hinweises auf die Patenterteilung: **14.05.2003**
(21) Anmeldenummer: 98119113.3
(22) Anmeldetag: 09.10.1998
(51) Int. Cl.: A61L 15/60, A61L 15/22

(54) **Verfahren zur Herstellung Superabsorber-Polymere enthaltender Körper und deren Verwendung**
Method for making a body containing a superabsorbent and use thereof
Procédé de préparation d'un corps contenant un polymère superabsorbant et son utilisation

(30) Priorität: 19.01.1998 DE 19801680
(43) Veröffentlichungstag der Anmeldung: 21.07.1999
(73) Patentinhaber: Carl Freudenberg KG, 69469 Weinheim (DE)
(72) Erfinder: Groitzsch, Dieter Dr., 69493 Hirschberg (DE); Schaut, Gerhard Dr., 69502 Hemsbach (DE)

(56) Entgegenhaltungen:
- WO-A-94/22502
- WO-A-95/31500
- US-A- 5 354 290

## Beschreibung

Die Erfindung betrifft ein Verfahren zur Herstellung von Superabsorber enthaltenden Körpern und deren deren Verwendung, insbesondere zur Herstellung von Windeln, Binden, Tampons und anderen Hygieneartikeln.

Absorbierende Strukturen, wie sie beispielsweise in Baby-Windeln, Erwachsenen-Windeln und der Damenhygiene eingesetzt werden, enthalten vorzugsweise sogenannte Superabsorber, um die Menge an absorbierender Körperflüssigkeit zu erhöhen, die Windeln dünner zu gestalten und die Körperflüssigkeiten chemisch in dem sich bildenden Gelkörper des Superabsorbers zu binden. Dadurch werden Leckagen verhindert.

Aus DE-OS 195 40 951 A1, DE-OS 196 07 551 A1, DE-PS 44 18 319 C2, US-PS 5 149 335, EP 0 453 286 A2 und WO 94/22502 A1 sind Hygieneartikel bekannt, die mit Polymerfasern verbundene Superabsorber-Polymere enthalten, welche als mikroporöser, offenzelliger Schwamm vorliegen.

In absorbierenden Strukturen ist in den letzten Jahren der Gehalt an SAP immer mehr erhöht worden mit folgenden Zielen: Die Dicke der absorbierenden Produkte sollte reduziert werden, und die Körperflüssigkeiten sollen so gebunden werden, daß sie auch bei mechanischer Beanspruchung (beim Tragen am Körper, Bewegung, Druck) nicht wieder abgegeben werden. Die Einführung von SAP in solchen Produkten hat sich hier als erfolgreich erwiesen, weil die Flüssigkeit mit SAP chemisch und nicht kapillar gebunden wird. Den Fasern oder Faserstoffen fällt dabei im wesentlichen der möglichst schnelle Flüssigkeitstransport zum SAP zu und dem SAP selbst die Funktion der Aufnahme der Flüssigkeit (Speicherfunktion). Mit besonderen konstruktiven Maßnahmen an der Windel, Damenbinde usw., hauptsächlich der rückseitigen Abdeckung mit einem dünnen, flüssigkeitsundurchlässigen Medium (Folie) und einer weiteren Abdeckung auf der dem Körper zugewandten Seite mit einer offenen Struktur, z.B. dünner, hydrophiler bzw. hydrophilierter Vliesstoff oder Folie mit vakuumexpandierten, dreidimensionalen Perforationen, Einsatz von hydrophobem, seitlichem Auslaufschutz und Einbau elastischer Elemente, gelingt es, das Problem von Leckagen weitgehend zu verhindern und die Haut trocken zu halten. Dem Vorteil des SAP, der druckbelastbaren und sehr hohen Fluidaufnahme, steht aber der Nachteil des Gelblockings und damit der Fluidtransportverlangsamung gegenüber. Die bekannten Maßnahmen, die Gelfestigkeit zu erhöhen bzw. den SAP-Partikeln eine Kern-/Mantel-Struktur zu verleihen, wobei der Kern stärker vernetzt ist als der Mantel, führen zu einer Herabsetzung der Fluidabsorption.

SAP (steht für Superabsorber-Polymer) ist in verschiedenen Ausführungsformen bekannt, wie Homofilfasern, die aus 100 % SAP bestehen, Bikomponentenfasern mit einem Kern aus nicht absorbierendem thermoplastischem Kunststoff und einem Mantel aus SAP sowie Puder oder Pulver.

SAP-Fasern lassen sich im Verschnitt mit anderen Fasern mit den bekannten trockenen Ablegetechniken zu einem fluidabsorbierenden Vliesstoff verarbeiten. Die einfache Verarbeitbarkeit spricht zwar für den Einsatz solcher SAP-bzw. anteilig SAP-haltiger Fasern, nachteilig ist jedoch deren relativ hoher Preis insbesondere im Vergleich zu Puder, Pulvern oder anderen Partikeln. Eine SAP-Stapelfaser auf Basis von Polyacrylsäure (bzw. deren Natriumsalz) hat etwa deren dreifachen Preis im Vergleich zu SAP in Pulverform. Deswegen haben sich solche Fasern insbesondere in Hygiene-Anwendungen nicht im großen Umfang durchsetzen können. Ganz besonders nachteilig erwiesen sich aus preislicher Sicht die Bikomponenten-Fasern, bei denen nur der Mantel aus SAP besteht. Der Mantelanteil macht nur 1/3 bis max. 1/2 der Gesamtfasermasse aus. Dadurch werden das Absorptionsvermögen entsprechend herabgesetzt und die Faserkosten in Relation zur Absorptionswirkung noch ungünstiger.

Aus SAP-Fasern können in Abmischung mit anderen Fasern mit bekannten trockenen Verfestigungsmethoden, wie beispielsweise mechanisches Vernadeln, flächiges oder musterförmiges Kalandern, Vliesstoffe gebildet werden, bei denen die Gefahr eines Ausrieselns von SAP-Partikeln im Vergleich zu SAP mit anderer Partikelform deutlich vermindert wird. Der Eintrag von SAP-Puder erfolgt entweder durch Aufstreuen auf ein dichtes Flächengebilde, wie z.B. Tissue-Papier, oder Einstreuen in eine offenporige, voluminöse Faserstruktur. Eine lose Ablage von SAP-Puder oder anderweitig geformter, nicht faseriger SAP-Partikel in einer absorbierenden Schicht ist nicht vorteilhaft. Die Position der Partikel kann sich während der Verwendung in ungewünschter Weise verändern. Die Partikel können im ungünstigsten Fall aus dem absorbierenden Produkt ausrieseln, sofern offenstrukturierte Oberflächen und/oder offene Ränder vorhanden sind.

Es existieren zahlreiche Lösungsvorschläge zur Fixierung der SAP-Partikel an den Fasern der absorbierenden Strukturen, d.h. entweder im Flächengebilde oder auf der Oberfläche des Flächengebildes.

Für die Lokalisierung der SAP-Partikel auf oder in dem absorbierenden Artikel werden diverse Methoden der feuchten Vorbehandlung des Flächengebildes vor SAP-Puderauftrag und auch trockene, d.h. thermische Fixierungsmethoden vorgeschlagen.
Aus EP-A-0 719 531 ist bekannt, daß das Trägermedium für den SAP mit Feuchte/Wasser vorbehandelt wird. Nach dem Auf- oder Eintragen der SAP-Partikel werden die wasserlöslichen Bestandteile des SAP (inhärente Anteile) zur Bindung aktiviert. Nach dem Trocknen sind die SAP-Partikel fest an die Fasern der absorbierenden Schicht gebunden.

In US 3,070,095 wird ein Verfahren beschrieben, bei dem SAP auf ein Tissue-Papier aufgestreut wird, anschließend mit einem zweiten Tissue-Papier abgedeckt wird und dann durch Pressen zwischen glatten Walzen zu einem Laminat verpreßt wird. Diese Methode hat allerdings den Nachteil, daß nur sehr wenig SAP aufgetragen werden kann, das SAP nicht mit Fasern durchsetzt ist und daher nur sehr schwach gebunden ist. Bei geringster mechanischer Beanspruchung rieselt SAP-Puder aus. Außerdem tendiert ein solches Laminat extrem zu Gelblocking, eine Eigenschaft, die für Hygiene-Anwendungen gefürchtet ist.

In WO 90/11181 und WO 91/10413 wird vorgeschlagen, Fasern mit einer wäßrigen Polymerdispersion anstelle von Wasser vorzubefeuchten. Nach der Trocknung kommen dann die Bindungskräfte sowohl des synthetischen Polymers der Dispersion als auch die inhärenten Bindungskräfte der SAP-Partikel zum Tragen.

In allen zuvor geschilderten Fällen sind die SAP-Partikel zwar an die Fasern bzw. in oder auf dem Flächengebilde gebunden. Die Trocknung bereitet sich jedoch schwierig und aufwendig, weil das in den SAP gebundene Wasser nur sehr widerwillig wieder abgegeben wird.

Ein großer Nachteil dieser SAP-Fixierungsmethoden ist die Tatsache, daß die Bindung nur im trockenen Zustand gewährleistet ist. Sobald sich nach Fluidadsorption aus dem SAP ein Gelkörper bzw. ein Hydrogel gebildet hat, fällt die Bindungskraft auf Null oder nahezu Null herab.

In EP A-0 720 488 wird eine trockene Methode zur Fixierung von SAP-Pulver in einem Vliesstoff als Trägermedium desselben beschrieben. Es werden Bindefasern, entweder aus einer Komponente (Homofilfasern) oder Bikomponenten-Fasern mit einem höher schmelzenden Kern und einem niedriger
schmelzenden/erweichenden Mantel vorgeschlagen. Durch Aufschmelzen der Bindefaser wird SAP an die Faser angebunden, aber auch in diesem Fall geht die Bindekraft nach der Fluidbeladung des SAP beinahe gänzlich verloren.

Es sind auch Lösungen vorgeschlagen worden, Fasern mit einem Superabsorberüberzug zu versehen. Entsprechend einer in US 4,721,647 beschriebenen Methode wird ein in Wasser gelöstes Monomer wie Acrylsäure, Methacrylsäure und Vinylsulfonsäure in Tropfenform auf die Faser eines absorbierenden Artikels appliziert und dort in Gegenwart von Initiatoren und Vernetzern zu einem Faserüberzug aus SAP vernetzt. Das Konzept beruht darauf, daß das SAP die Faser fest umschließt und selbst nach dem Quellen zu einem hydrogelartigen Zustand fest mit der Faser verbunden ist.

Alle oben beschriebenen Methoden der Applikation von SAP über die wasserlöslichen Vorstufen oder olefinisch ungesättigten Monomeren führen zu einer deutlichen Verhärtung in einem Vliesstoff im Vergleich zu seinem Zustand vor der SAP-Applikation und zum Teil zu einer deutlich niedrigeren Auflage als beim Einstreuen von SAP-Pulver.

Aus WO 95/31500 A2 und US 5,328,935 sind SAP-Schäume und Verfahren zu ihrer Herstellung bekannt.

Gemäß US 5,328,935 wird der Superabsorber-Polymerschaum aus einem im Wesentlichen wasserlöslichen, ungesättigten Monomer, das neutralisierte Carboxylgruppen aufweist, und aus einem im Wesentlichen wasserlöslichen, inerten Vernetzer hergestellt. Das Monomer und der Vernetzer werden in Gegenwart eines im Wesentlichen wasserunlöslichen Treibmittels eines geeigneten Lösungsmittels expandiert und umgesetzt, um einen SAP-Schaum zu erhalten, der im Wesentlichen durchgängig miteinander verbundene Kanäle aufweist und ein relativ hohes Verhältnis von Oberfläche zu Masse besitzt.

Gemäß WO 95/31500 A2 werden ein vernetzbares Polymer und ein erstes Lösungsmittel zu einer stabilen Lösung vermischt, welche einer Phasenseparation unterworfen wird, bei der eine mit Polymer angereicherte Phase und eine Lösungsmittelphase entstehen. Die Polymerphase wird während der Phasentrennung zum Vernetzen angeregt; es entsteht ein mikroporöser Schaum, der anschließend getrocknet wird.

In beiden vorgenannten Verfahren des Standes der Technik werden Schaumvorläufer in einer Form umgesetzt, so dass ein geformter, fester Polymerschaum entsteht. Dieser erhält seinen strukturellen Zusammenhalt aus dem SAP selbst, so dass die Menge des im Schaum vorliegenden - teuren - Superabsorbers hoch sein muß.

Aufgabe der vorliegenden Erfindung ist die Bereitstellung eines Verfahrens zur Herstellung von SAP enthaltenden Körpern, im folgenden auch als "absorbierende Strukturen" bezeichnet, das die Nachteile der bekannten Strukturen sowie eine Verhärtung im Vliesstoff und das Gelblocking bei der Anwendung vermeidet und zu einem Produkt führt, das die aufgenommene Flüssigkeiten gut zurückhält. Dabei soll die Menge des im Körper enthaltenen, kostenintensiven Superabsorbers je nach Anforderung auch mengenmäßig nach unten variiert werden können, ohne dass der strukturelle Zusammenhalt gefährdet ist.

### Darstellung der Erfindung

Die Aufgabe wird erfindungsgemäß gelöst durch ein Verfahren mit den kennzeichnenden Merkmalen des ersten Patentanspruchs. Vorteilhafte Varianten sowie die Verwendung des nach diesem Verfahren hergestellten Körpers sind in den Unteransprüchen aufgezeigt.

Erfindungsgemäß wird ein Superabsorber-Schaum nach einem an sich aus WO 95/31500 A2 bekannten Verfahren hergestellt. Zu Beginn der Herstellung werden jedoch Polymerfasern, insbesondere ein vorgebundener Vliesstoff oder ein loser Faserflor, in das Reaktionsgemisch eingebracht, so dass letztendlich ein Superabsorber-Polymer enthaltender Körper erhalten wird.

Mit dem erfindungsgemäßen Verfahren hergestellte, SAP-haltige Körper weisen die erwähnten Nachteile nicht oder in verringertem Maße auf, weil die SAP-Partikel eine besondere mikroporöse, offenzellige Struktur besitzen und in dieser Struktur in dem absorbierenden Produkt verteilt sind.

Das erfindungsgemäße, nachstehend eingehend beschriebene Verfahren zur Herstellung des absorbierenden Körpers verlangt den Einsatz von wasserlöslichen polymeren oder präpolymeren Substanzen mit reaktiven, vernetzbaren Gruppen. Typische funktionelle Gruppen, die leicht mit geeigneten Vernetzern in Reaktion gebracht werden können, sind Hydroxyl-, Amino- und Carboxylgruppen. Die wasserlöslichen Polymere verlieren durch die Quervemetzung ihre Wasserlöslichkeit und gehen in einen in Wasser unlöslichen aber quellbaren Zustand über.

Solche wasserlöslichen Polymere sind beispielsweise hydroxyalkylierte Stärken, hydroxyalkyliertes Guar und hydroxyalkyliertes Dextran, Copolymere aus Vinylalkohol und Vinylacetat, Polymethacrylsäure, Acrylat- bzw. Methacrylat-Copolymere, wie Poly(hydroxypropylacrylat-co-acrylamid),
Poly(hydroxyethylacrylat-co-diacetonacrylamid) und Poly(hydroxypropylacrylatco-hydroxyethylacrylat.

Vorzugsweise ist das SAP ausgewählt aus Hydroxypropyldextran, Hydroxypropylguar, Hydroxypropylstärke, Hydroxypropylcellulose, Hydroxyethylcellulose, Methylcellulose, Hydroxypropylmethylcellulose oder Ethylhydroxyethylcellulose.

Geeignete Polypeptide sind beispielsweise Poly(L-prolin) und Poly(valin-prolinlycin-X-glycin) mit X = beliebige Aminosäure.

Besonders bevorzugt werden Hydroxypropylcellulose oder Hydroxyethylcellulose, speziell Hydroxpropylcellulose, eingesetzt.
Im fertigen, SAP enthaltenden Körper sind die SAP vernetzt. Geeignete Vernetzer sind beispielsweise Acetaldehyd, Formaldehyd, Glutaraldehyd, Diglycidylether, Divinylsulfon, Diisocyanat, Dimethylharnstoff, Epichlorhydrin, Oxalsäure, Phosphorylchloride, Trimetaphosphat, Trimethylolmelamin, polyacroleinähnliche Verbindungen. Bevorzugt sind Formaldehyd, Glutaraldehyd, Divinylsulfon und Epichlorhydrin.

Das SAP kann im Prinzip aus solchen Polymeren bestehen, die mindestens das 20-fache des eigenen Trockengewichtes an Flüssigkeit aufnehmen und dabei kein Hydrogel bilden. Das Superabsorber-Polymer ist vorzugsweise ausgewählt aus hydrophob modifizierten Kohlenwasserstoffpolymeren, Poly(vinylalkohol-covinylacetat), Poly(meth)acrylsäure, cyanoethyliertem oder teilweise formyliertem Poly(vinylalkohol), Poly-N-vinyl-2-oxazolidon, Polypep-tiden, (Meth)acrylat-Copolymeren und N-Alkyl(meth)acrylamid-Derivaten. Solche Polymere schließen Salze der Polyacrylsäure oder Copolymere ein, die durch Copolymerisation von Acrylsäure und Comonomeren wie Maleinsäure, Itaconsäure, Acrylamid, 2-Methylpropansulfonsäure, 2-Methacrylethansulfonsäure, 2-Hydroxyethylmethacrylat oder Styrolsulfonsäure, bei einem Copolymerisationsverhältnis, das die Eigenschaften des SAP nicht negativ beeinflußt, enthalten werden.

Als Polymerfasern können erfindungsgemäß alle geeigneten Polymerfasern eingesetzt werden. Beispiele geeigneter Fasern sind Polyethylenfasern, Polypropylenfasern, Polyethylenterephthalat-Fasern und weitere Fasern, die üblicherweise zur Herstellung von Windeln und ähnlichen Produkten eingesetzt werden.

Die Körper werden hergestellt durch:
a) Vermischen eines vernetzbaren Superabsorber-Polymers mit einem Lösungsmittel zur Ausbildung einer Superabsorber-Polymer-Lösung, die phasensepariert wird, und Tränken von Polymerfasern mit dieser Lösung,
b) Induzieren einer Phasenseparation der Superabsorber-Polymer-Lösung in eine faserhaltige, Polymer-angereicherte Phase und eine Polymer-abgereicherte Phase,
c) Vernetzen des Superabsorber-Polymers in der faserhaltigen, Polymer-angereicherten Phase während der Phasenseparation zur Ausbildung eines mikroporösen, offenzelligen Schaums an den Fasern, und
d) Trocknen des entstandenen Superabsorber-Polymer enthaltenden Flächengebildes unter Erhalt der mikroporösen, offenzelligen Struktur im Superabsorber-Polymer.

Es wird also im Prinzip dem Verfahrensschema zur Herstellung von SAP-Schäumen, die jedoch nicht mit Polymerfasern in Kontakt stehen, aus WO 95/31500 A 2 gefolgt.

Das Induzieren der Phasenseparation wird dabei durch Aufheizen auf eine Temperatur im Bereich bis zu 98°C, gegebenenfalls unter Wasserdampfatmosphäre, oder durch Zusatz von flüchtigen Nicht-Lösungsmitteln für das SAP erreicht.

Generell wird die Lösung eines vernetzbaren Polymers durch Ingangsetzen der Vernetzung und ggfs. durch Zusatz phasentrennungsbeschleunigender Agenzien zu einer Phasentrennung zu bewegen. In der einen Phase verarmt das Polymer, in der anderen wird Polymer angereichert. In der polymerangereicherten Phase wird die Vernetzung weitergeführt, bis sich durch Koagulation ein mikroporöser Schaumstoff gebildet hat. Schließlich wird getrocknet. Der Prozeß läuft bis zur Stufe der Porenbildung, vorzugsweise in einer vorgefertigten, hermetisch abgeriegelten Form, ab. Zur Trocknung wird der nasse Schwamm aus der Form herausgeholt.

Vorteilhafterweise wird ein bereits vorgebundener Vliesstoff oder ein loser Faserflor nach der Applikation einer vernetzbaren, wäßrigen Polymerlösung, die mit einem Vernetzer versetzt worden ist, unter Wasserdampfatmosphäre langsam auf 98°C aufgeheizt, die Vernetzung und die damit einhergehende Ausfällung der Mikroporösstruktur bereits während der Aufheizperiode in Gang gesetzt und nach erreichter Endtemperatur ebenfalls unter Wasserdampfatmosphäre zu Ende geführt. Die Separation des SAP kann dabei durch Zusatz von Elektrolyten oder anderen bekannten Nichtlösern für das in Wasser gelöste Polymer beschleunigt werden. Nach vollständiger Ausfällung der Vernetzung wird getrocknet. Zur Trocknungsbeschleunigung kann zuvor ein an sich bekannter Lösungsmittelaustausch vorgenommen werden, in der Weise, daß Wasser in den Mikroporen zuerst durch ein erstes wasserlösliches Lösungsmittel wie Ethanol verdrängt wird und dieses wiederum durch ein zweites, im Vergleich zum ersten Lösungsmittel sehr tief siedendes Lösungsmittel, z.B. Heptan, verdrängt wird.

Die weiteren Verfahrensparameter können der WO 95/31500 A2 entnommen werden. Das Aufheizen der Polymerlösung erfolgt vorzugsweise bei einer Temperatur oberhalb der unteren Entmischungstemperatur. Genaue Angaben über die untere Entmischungstemperatur, wie auch die Konzentrationen an Polymer und Vernetzer in der Lösung, sind dieser Schrift zu entnehmen. Auch
die Angaben zu den angewendeten Reaktionszeiten und Temperaturverläufen können der WO 95/31500 entnommen werden.

Die erfindungsgemäß hergestellte, SAP enthaltenden Körper oder Multischicht-Körper werden vorzugsweise zur Herstellung von Windeln, Binden, Tampons oder anderen Hygieneartikeln eingesetzt.

Die entstandene Struktur kann auch als mikroporöse Schwammstoff-Struktur bezeichnet werden. Unter Partikel verstehen wir hier jede Art der Anlagerungsund Verteilungsform in dem absorbierenden Produkt. Das mikroporöse SAP kann die Fasern gleichmäßig umschlingen, einen perlenartigen Überzug über die Fasern des absorbierenden Produktes bilden, eine vollig ungleichmäßige Verteilung um die Faser und zwischen den Fasern aufweisen und/oder an den Faserkreuzungspunkten sitzen und dort die Funktion einer Bindungssubstanz für die Fasern übernehmen. Die Polymerfasem können in einer Fertigungsvorstufe, insbesondere vor dem SAP-Eintrag, bereits eine ausreichende Integrität aufweisen, so daß eine weitere Integritätserhöhung nicht notwendig
ist, aber gewünscht sein kann. Die Vorstufe des absorbierenden Produktes vor der SAP-Applikation kann also ein Vliesstoff sein, der durch bekannte Vlieslegemethoden und bekannte Verfestigungsmethoden eine für die Endanwendung ausreichende Integrität aufweist.

Es können aber auch Flore oder Blätter aus losen ungebundenen Fasern für die SAP-Applikation eingesetzt werden. Solche Flore oder Blätter können ungestützt nicht transportiert oder anderweitig gehandhabt oder weiterverarbeitet werden. In diesem Fall sind der mikroporöse Schaum bzw. die mikroporösen SAP-Partikel in ihrer gesamten Zahl oder einem Teil der Gesamtzahl als Bindemittel für die Integrität des absorbierenden Produktes alleine oder überwiegend verantwortlich.

Die Schaumstruktur der SAP-Partikel ist offenzellig oder überwiegend offenzellig. Darunter ist die Tatsache zu verstehen, daß die Poren, die in dem SAP eingelassen sind, miteinander bis zu 100% in Verbindung stehen. Aufgrund der durchgehenden Mikrokanäle innerhalb der SAP-Partikel in dem absorbierenden Produkt wird die Flüssigkeit schneller in den Kern desselben transportiert als bei herkömmlichen SAP-Kompaktmassen. Aufgrund der starken Hydrophilie der mikroporösen SAP-Struktur werden wäßrige Medien trotz der geringen Porengröße sehr schnell in den Kern des Partikels transportiert. Die Porengröße liegt im Bereich von 0,2 bis 100 µm, vorzugsweise 0,5 bis 50 µm.

In und auf dem SAP können Netzmittel oder andere Surfactants (Tenside) appliziert bzw. integriert sein, die die Grenzflächenspannung von SAP-Polymer zu Wasser noch weiter senken. Dadurch wird der Flüssigkeitstransport sowohl im Mikrobereich, d.h. innerhalb des SAP, als auch im Makrobereich, d.h. im gesamten Volumen des absorbierenden Produktes, beschleunigt.

Der erfindungsgemäß hergestellte, absorbierende Körper kann direkt verwendet werden oder eine Komponente eines absorbierenden Produktes mit Multischichtstruktur sein. Solche Multischicht-Produkte werden z.B. in Baby-Windeln, Erwachsenen-Windeln und Damenbinden eingesetzt. Es ist bekannt, daß zwischen einem Topsheet (Abdechvliesstoff) und einer rückseitig flüssigkeitsdichten Folie ein absorbierender Kern plaziert ist. Zwischen dem Kern und dem Topsheet kann eine weitere Schicht zwischengeschoben sein, die die Körperflüssigkeit spontan aufnimmt und zeitlich verschoben in den Kern weiterleitet zur endgültigen Speicherung hauptsächlich im SAP und teilweise in den interkapillaren Räumen zwischen den Fasern. Ein Gelblocking kann durch diese Zwischenschicht weitgehend vermieden werden.

Ein derartiger Multischicht-Körper enthält in dieser Reihenfolge
1) ein flüssigkeitsdurchlässiges Topsheet,
2) eine flächige Schicht eines mit dem erfindungsgemäßen Verfahren hergestellten Flächengebildes und
3) eine flüssigkeitsundurchlässige Schicht.

Der Superabsorber-Polymere enthaltende Körper kann jede beliebige Form aufweisen. Vorzugsweise bleibt er bei Verwendung in Windeln, Binden oder Slipeinlagen in flächiger Form. Bei Verwendung in Tampons kann er auch beispielsweise in zylindrischer Form zu einem Raumkörper verformt werden.

Das SAP liegt zumindest teilweise als mikroporöser offenzelliger Schaum vor. Dabei können noch andere SAP-Partikel vorliegen, die kein Schaum sind. Vorzugsweise liegt jedoch im wesentlichen das gesamte SAP bzw. das gesamte SAP als mikroporöser offenzelliger Schaum vor.

Die Verteilung des schwammartigen mikroporösen SAP kann über den gesamten Querschnitt des Körpers verteilt sein. Seine Verteilung kann sich aber auch nur auf einen Teil des Querschnittes beschränken, so daß eine Seite völlig frei von SAP ist. Es kann auf einer Seite konzentrierter als auf der anderen Seite vorliegen, so daß ein Verteilungsgradient senkrecht zu der Fläche von einer Oberfläche zur anderen vorliegt. Die Seite mit der höheren offenzelligen SAP-Partikeldichte ist dann vorzugsweise dem Kern bzw. der rückseitigen flüssigkeitsundurchlässigen Folienabdeckung zugewandt.

In das SAP mit mikroporöser Schaumstoffstruktur können auch völlig porenfreie SAP-Partikel eingelagert und/oder angelagert oder in irgend einer anderen Weise in oder auf der Mikroporösstruktur verteilt sein. Dies kann dann von Vorteil sein, wenn es weniger darauf ankommt, das SAP zum schnellen Quellen, d.h. stark beschleunigter Fluidaufnahme zu veranlassen, sondern wenn es aus anwendungstechnischen Gründen angebracht ist, die Menge der superabsorbierenden Substanz pro Volumeneinheit des absorbierenden Produktes insgesamt zu erhöhen.

Das mit dem erfindungsgemäßen Verfahren hergestellte Produkt zeichnet sich außer einer stark beschleunigten Fluidaufnahme im Mikrobereich des SAP zusätzlich durch eine extreme Weichheit, Anschmiegbarkeit und Verformbarkeit aus. Diese Eigenschaften sind in absorbierenden Produkten wie insbesondere Windeln besonders geschätzt, um Leckagen nach erfolgter Abgabe der Körperflüssigkeit zu verhindern. Die Anpassung an Körperformen erfolgt mit solchen Produkten in Kombination mit Elastics (elastisden Komponenten) besser als mit steiferen, weniger nachgebenden und härter gebundenen Produkten.

## Patentansprüche

1. Verfahren zur Herstellung von Superabsorber enthaltenden Körpern **gekennzeichnet durch**
a) vermischen eines vernetzbaren Superabsorber-Polymers mit einem Lösungsmittel zur Ausbildung einer Superabsorber-Polymer-Lösung, die phasenseparierbar ist, und Tränken von Polymerfasern mit dieser Lösung,
b) Induzieren einer Phasenseparation der Superabsorber-Polymer-Lösung in eine faserhaltige Polymer-angereicherte Phase und eine Polymer-abgereicherte Phase,
c) Vernetzen des Superabsorber-Polymers in der faserhaltigen Polymer-angereicherten Phase während der Phasenseparation zur Ausbildung eines mikroporösen, offenzelligen Schaums an den Fasern, und
d) Trocknen des entstandenen Superabsorber-Polymer enthaltenen Flächengebildes unter Erhalt der mikroporösen offenzelligen Struktur im Superabsorber-Polymer.

2. Verfahren nach Anspruch 1, **dadurch gekennzeichnet, daß** das Induzieren der Phasenseparation durch Aufheizen auf eine Temperatur im Bereich bis zu 98°C, gegebenenfalls unter Wasserdampfatmosphäre, oder durch Zusatz von flüssigen Nicht-Lösungsmitteln für das Superabsorber-Polymer erfolgt.

3. Verfahren nach Anspruch 1 oder 2, **dadurch gekennzeichnet, daß** die Polymerfasern als vorgebundener Vliesstoff eingesetzt werden.

4. Verfahren nach Anspruch 1 oder 2, **dadurch gekennzeichnet, daß** die Polymerfasern als loser Faserflor eingesetzt werden.

5. Verwendung von Körpern, hergestellt nach einem der Ansprüche 1 bis 4, zur Herstellung von gegebenenfalls mehrschichtigen Windeln, Binden, Tampons und anderen Hygieneartikeln.

## Claims

1. A process for preparing superabsorbent-containing bodies, **characterized by**
a) mixing a crosslinkable superabsorbent polymer (SAP) with a solvent to form a superabsorbent polymer solution which can be induced to phase separate, and saturating polymeric fibres with this solution,
b) inducing the superabsorbent polymer solution to phase separate into a fibre-containing polymer-concentrated phase and a polymer-dilute phase,
c) crosslinking the superabsorbent polymer in the fibre-containing polymer-concentrated phase during the phase separation to form a microporous, open-celled foam on the fibres, and
d) drying the resulting SAP-containing body to obtain the microporous open-celled structure in the superabsorbent polymer.

2. A process according to claim 1, **characterized in that** the inducing of the phase separation is effected by heating to a temperature in the range up to 98°C in the presence or absence of a water vapour atmosphere or by adding liquid nonsolvents for the superabsorbent polymer.

3. A process according to claim 1 or 2, **characterized in that** the polymeric fibres are used in the form of prebound nonwoven fabric.

4. A process according to claim 1 or 2, **characterized in that** the polymeric fibres are used in the form of loose fibre web.

5. Use of bodies produced according to any one of claims 1 to 4 for producing single- or multilayered diapers, napkins, tampons and other hygiene articles.

## Revendications

1. Procédé pour la préparation de corps contenant un superabsorbant **caractérisé par**
a) le mélange d'un polymère superabsorbant réticulable avec un solvant pour former une solution de polymère superabsorbant, qui serait séparable par phases et immersion de fibres de polymère avec cette solution,
b) l'induction d'une séparation des phases de la solution de polymère superabsorbant en une phase enrichie en polymère contenant des fibres et une phase appauvrie en polymère,
c) la réticulation du polymère superabsorbant dans la phase enrichie en polymère contenant des fibres pendant la séparation des phases afin de former une mousse microporeuse, à cellules ouvertes sur les fibres et
d) le séchage de la structure plane formée contenant le polymère superabsorbant en maintenant la structure microporeuse, à cellules ouvertes dans le polymère superabsorbant.

2. Procédé selon la revendication 1, **caractérisé en ce que** l'induction de la séparation des phases est réalisée par chauffage à une température dans la plage jusqu'à 98 °C, le cas échéant sous une atmosphère de vapeur d'eau ou par addition de non-solvants liquides pour le polymère superabsorbant.

3. Procédé selon la revendication 1 ou 2, **caractérisé en ce que** les fibres de polymère sont utilisées sous forme d'un non-tissé de fibres liées au préalable.

4. Procédé selon la revendication 1 ou 2, **caractérisé en ce que** les fibres de polymère sont utilisées sous forme de voile de carde délié.

5. Utilisation de corps préparés selon l'une quelconque des revendications 1 à 4 pour la production de couches, bandes, tampons et autres articles hygiéniques, le cas échéant à plusieurs couches.
